Europäisches Patentamt

European Patent Office

Office européen des brevets

(19)

(11) **EP 1 368 007 B1**

(12) **EUROPÄISCHE PATENTSCHRIFT**

(45) Veröffentlichungstag und Bekanntmachung des Hinweises auf die Patenterteilung:
**05.10.2005 Patentblatt 2005/40**

(21) Anmeldenummer: 01913827.0

(22) Anmeldetag: **27.02.2001**

(51) Int Cl.$^7$: **A61K 9/28**

(86) Internationale Anmeldenummer:
**PCT/EP2001/002018**

(87) Internationale Veröffentlichungsnummer:
**WO 2002/067906 (06.09.2002 Gazette 2002/36)**

(54) **ÜBERZUGS- UND BINDEMITTEL FÜR ARZNEIMITTELFORMULIERUNGEN MIT VERBESSERTER LAGERSTABILITÄT**

COATING AND BINDING AGENT FOR PHARMACEUTICAL FORMULATIONS WITH IMPROVED STORAGE STABILITY

AGENT D'ENROBAGE ET LIANT POUR COMPOSITIONS PHARMACEUTIQUES A STABILITE AMELIOREE EN CAS DE STOCKAGE

(84) Benannte Vertragsstaaten:
**AT BE CH CY DE DK ES FI FR GB GR IE IT LI LU MC NL PT SE TR**

(43) Veröffentlichungstag der Anmeldung:
**10.12.2003 Patentblatt 2003/50**

(73) Patentinhaber: **Röhm GmbH & Co. KG**
**64293 Darmstadt (DE)**

(72) Erfinder:
• **PETEREIT, Hans-Ulrich**
**64291 Darmstadt (DE)**
• **MEIER, Christian**
**64295 Darmstadt (DE)**
• **ROTH, Erna**
**64297 Darmstadt (DE)**

(56) Entgegenhaltungen:
**DE-A- 19 918 435**

• **DATABASE WPI Section Ch, Week 199952 Derwent Publications Ltd., London, GB; Class A96, AN 1993-348343 XP002184934 & JP 02 973751 B (TAISHO PHARM CO LTD), 8. November 1999 (1999-11-08)**

**Beschreibung**

Die Erfindung betrifft ein Überzugs- und Bindemittel für Arzneimittelformulierungen mit verbesserter Lagerstabilität.

Stand der Technik

WO 00/05307 beschreibt ein Verfahren zur Herstellung eines Überzugs- und Bindemittels für orale oder dermale Arzneiformen bestehend aus (a) 35 - 98 Gew.-% eines Copolymers, bestehend aus radikalisch polymerisierten C1- bis C4-Estern der Acryl- oder Methacrylsäure und weiteren (Meth)acrylatMonomeren, die funktionelle tertiäre Ammoniumgruppen aufweisen und (b) 1 - 50 Gew.-% eines Weichmachers sowie 1 - 15 Gew.-%, eines Emulgators mit einem HLB-Wert von mindestens 14 wobei die Komponenten (a), (b) und (c) mit oder ohne Zusatz von Wasser und gegebenenfalls unter Zusatz eines pharmazeutischen Wirkstoffs und weiterer üblicher Zuschlagstoffe miteinander vermengt werden und das Überzugs- und Bindemittel durch Schmelzen, Gießen, Ausstreichen oder Aufsprühen hergestellt wird, wobei das Copolymer (a) in Pulverform mit einer mittleren Teilchengröße von 1 - 40 µm eingebracht wird.
Die Formulierung in der definierten Pulverform in Kombination mit Weichmacher und Emulgator ermöglicht es, die entsprechenden Copolymere ohne den Zusatz von Säuren in stabile wäßrige Lösungen bzw. Dispersionen zu überführen. Es besteht der Vorteil, daß ein ansonsten auftretender bitterer Eigengeschmack des Überzugsmittels vermieden werden kann. Die Überzugs- und Bindemittel sind zudem in Wasser kaum löslich, lösen sich aber in künstlichem Magensaft rasch auf. Sie eigenen sich daher insbesondere für geschmacksisolierende, im Magensaft rasch zerfallende Formulierungen. Zur Wasserdampfdurchlässigkeit der Überzüge sind kein Angaben enthalten. Aufgabe und Lösung
Eine ganze Reihe von pharmazeutischen Wirkstoffen sind zwar an trockener Luft sehr stabil, jedoch empfindlich gegenüber Feuchtigkeit und feuchtigkeitsbedingten pH-Verschiebungen in den alkalischen pH-Bereich hinein. Nicht überall sind die Lagerungsbedingungen für überzogene oder gebundene Arzneimittelformulierungen optimal, so daß es z. B. in tropischen Regionen durchaus vorkommen kann, daß solche Arzneimittelformulierungen vor Gebrauch über längere Zeit relativ hohen Luftfeuchtigkeiten ausgesetzt sind. Es ist daher generell von Bedeutung, daß möglichst wenig Feuchte durch die Arzneimittelüberzüge oder Bindemittel zum eingeschlossenen Wirkstoff vordringen kann. Es wurde daher als Aufgabe gesehen Überzugs- und Bindemittels für orale oder dermale Arzneiformen bereitzustellen, die eine gute Isolierung gegenüber dem Eindringen von Luftfeuchte gewährleisten.
Die Erfindung geht im wesentlichen von der WO 00/05307 aus. Die dort beschriebenen Überzugs- und Bindemittel sollten insbesondere dahingehend weiterentwickelt werden, daß ihre Wasserdampfdurchlässigkeit verbessert wird, ohne daß die übrigen Eigenschaften wie rascher Zerfall in künstlichem Magensaft, gute Verarbeitbarkeit beeinträchtigt werden.
Die Aufgabe wird gelöst durch ein
Verfahren zur Herstellung eines Überzugs- und Bindemittels für orale oder dermale Arzneiformen bestehend im wesentlichen aus

(a) einem Copolymer, bestehend aus radikalisch polymerisierten C1- bis C4-Estern der Acryl- oder Methacrylsäure und weiteren (Meth)acrylat-Monomeren die funktionelle tertiäre Ammoniumgruppen aufweisen, wobei das Copolymer in Pulverform mit einer mittleren Teilchengröße von 1 - 40 µm vorliegt

(b) 3 bis 15 Gew.-%, bezogen auf (a), eines Emulgators mit einem HLB-Wert von mindestens 14

(c) 5 bis 50 Gew.-%, bezogen auf (a), einer $C_{12}$- bis $C_{18}$-Monocarbonsäure oder einer $C_{12}$- bis $C_{18}$-Hydroxylverbindung

wobei die Komponenten (a), (b) und (c) mit oder ohne Zusatz von Wasser und gegebenenfalls unter Zusatz eines pharmazeutischen Wirkstoffs und weiterer üblicher Zuschlagstoffe miteinander vermengt oder vermischt werden und das Überzugs- und Bindemittel aus der Mischung durch Schmelzen, Gießen, Ausstreichen, Aufsprühen oder Granulieren hergestellt wird.
Während Überzugs- und Bindemittel nach WO 00/05307 Wasserdampfdurchlässigkeiten gemessen nach DIN 53 122 im Bereich von 400 $(g/m^2/d)$ oder darüber weisen, liegen die erfindungsgemäßen Überzugs- und Bindemittel bei Wasserdampfdurchlässigkeiten von höchstens 350 $(g/m^2/d)$, bevorzugt höchtens 300 $(g/m^2/d)$, besonders bevorzugt höchstens 200 $(g/m^2/d)$. Es war nicht vorhersehbar, daß die sich dieser Effekt durch die Kombination der Komponenten (a), (b) bis (c) erreichen lassen würde, ohne daß die bekannten vorteilhaften Eigenschaften von Überzugs- und Bindemittel nach WO 00/05307 beeinträchtigt werden. Insbesondere ermöglicht die Verwendung der Komponente (c) auch einen weitgehenden oder völligen Verzicht auf übliche Weichmacher. Dies ist ein weiterer Vorteil, da man stets bemüht ist, die Zahl der Komponenten bei Arzneimittelformulierungen gering zu halten.

Ausführung der Erfindung

Komponente (a)

Die Copolymere (a) bestehen im wesentlichen oder ganz aus radikalisch polymerisierten C1- bis C4-Estern der Acryl- oder Methacrylsäure und weiteren (Meth)acrylat-Monomeren, die funktionelle tertiäre Ammoniumgruppen aufweisen.

Geeignete Monomere mit funktionellen tertiäre Ammoniumgruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat. Besonders bevorzugt ist Dimethylaminoethylmethacrylat.

Der Gehalt der Monomere mit tertiären Ammoniumgruppen im Copolymeren kann vorteilhafterweise zwischen 30 und 70 Gew.-%, bevorzugt zwischen 40 und 60 Gew.-% liegen. Der Anteile der C1- bis C4-Estern der Acryl- oder Methacrylsäure beträgt 70 - 30 Gew.-%. Zu nennen sind Methylmethacrylat, Ethylmethacrylat, Butylmethacrylat, Methylacrylat, Ethylacrylat, Butylacrylat.

Ein der Komponente (a) entsprechendes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein. Der Anteil der Komponente (a) an der Formulierung beträgt bevorzugt 50 - 90 Gew.-%.

Die Copolymere (a) werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

Die mittlere Teilchengröße der Pulver kann wie folgt bestimmt werden:

- Durch Luftstrahlsiebung zur einfachen Aufteilung des Mahlproduktes in wenige Fraktionen. Diese Methode ist in diesem Meßbereich etwas ungenauer als die Alternativen. Mindestens 70, bevorzugt 90 % der Teilchen bezogen auf die Masse (Masseverteilung) sollen jedoch in dem erfindungsgemäßen Größenbereich von 1 - 40, bevorzugt 10 - 30 µm liegen.

- Eine gut geeignete Meßmethode ist die Laserbeugung zur Bestimmung der Korngrößenverteilung. Handelsübliche Geräte erlauben die Messung in Luft (Fa. Malvern S3.01 Partikelsizer) oder bevorzugt in flüssigen Medien (Fa. LOT, Galai CIS 1). Voraussetzung für die Messung in Flüssigkeiten ist, das sich das Polymer darin nicht löst oder die Teilchen auf eine andere Weise während der Messung verändern. Ein geeignetes Medium ist z. B. eine stark verdünnte (ca. 0,02%ige) wäßrige Polysorbat 80 Lösung. Der mittlere Teilchendurchmesser muß im Bereich zwischen 1 und 40, bevorzugt zwischen 5 und 35, insbesondere zwischen 10 und 20 µm liegen.

Komponente (b)

Emulgatoren oder Tenside sind grenzflächenaktive Substanzen mit lyobipolarem Charakter, d.h. in ihrem Molekül müssen unpolare, lipophile und polare, hydrophile Zentren vorliegen (P.H. List, Arzneiformenlehre, Wissenschaftliche Verlagsgesellschaft mbH Stuttgart,1982,Kap. 6.2.). Je nach molekularem Aufbau unterscheidet man zwischen ionogenen und nichtionogen Emulgatoren.

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)).

Ein HLB-Wert (Hydrophile/Lipophile Balance) läßt sich nur bei nicht ionischen Emulgatoren exakt bestimmen. Bei anionischen Emulgatoren kann dieser Wert rechnerisch ermittelt werden, liegt jedoch praktisch immer über oder weit über 14.

Unter Emulgatoren (b) mit einem HLB-Wert über 14 werden erfindungsgemäß hydrophile, nicht ionische Emulgatoren mit HLB - Bereich von mindestens 14 sowie ebenfalls hydrophile, anionische Emulgatoren und deren Salze, die einen rechnerischen HLB-Wert über 14 aufweisen, verstanden. Emulgatoren mit HLB-Werten von weniger als 14, wie z. B. Glycerolmonostearat können zwar zusätzlich ebenfalls enthalten sein, ersetzen jedoch die Emulgatoren (b) mit HLB-Werten von mindestens 14 nicht.

Geeignete Emulgatoren (b) sind z. B. Natriumlaurylsulfat und Natriumcetylstearylsulfat, Saccharosestearat und Polysorbat 80.

Die Emulatoren (b) sind in Mengen von 1 - 15, bevorzugt 5 - 10 Gew.-% bezogen auf die Komponente (a) enthalten. Möglich ist natürlich auch der Einsatz von Emulgatormischungen.

Die Zugabe der Emulgatoren (b) zur Formulierung kann in bekannter Weise, direkt, in wäßriger Lösung oder nach thermischer Vorbehandlung der Mischung vorgenommen werden.

Die Emulgatoren können je nach Typ (lipophil oder hydrophil) und zugesetzter Menge die Funktionalität der Polymerschicht beeinflussen.

Komponente (c)

Komponente (c): 5 bis 50, bevorzugt 10 bis 20 Gew.-% (bezogen auf die Komponente (a)) einer $C_{12}$- bis $C_{18}$-Monocarbonsäure oder einer $C_{12}$- bis $C_{18}$-Hydroxylverbindung. Die Komponente (c) ist entscheidend für die überraschend geringe Wasserdampfdurchlässigkeit der Formulierungen.

Bevorzugt sind unverzweigte $C_{12}$- bis $C_{18}$-Monocarbonsdure oder einer $C_{12}$- bis $C_{18}$-Hydroxylverbindungen. Es können gegebenenfalls auch verzweigte Derivate der genannten Substanzen geeignet sein.

$C_{12}$- bis $C_{18}$-Monocarbonsäuren sind z.B insbesondere Laurinsäure und Myristinsäure. Bevorzugt sind Palmitinsäure und Stearinsäure.

$C_{12}$- bis $C_{18}$-Hydroxylverbindung, insbesondere Alkanole mit einer endständigen Hydroxylgruppe wie z. B. Laurylalkohol oder Stearylalkohol.

Weitere Zuschlagstoffe

Der erfindungsgemäßen Formulierung werden in der Regel bei der Verarbeitung zu Überzugs- und Bindemittels übliche Zuschlagstoffe hinzugefügt.

Einsatzmengen und Verwendung der üblichen Zuschlagstoffe in Arzmeimittelüberzügen oder Beschichtungen sind dem Fachmann geläufig. Übliche Zuschlagstoffe können z. B. Trennmittel, Pigmente, Stabilisatoren, Antioxidantien, Porenbildner, Penetrationsförderer, Glanzmittel, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

• Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemittel liegen zwischen 0,5 bis 100 Gew.-% bezogen auf das Copolymer (a).

In einer besonders vorteilhaften Ausführungsform erfolgt der Zusatz des Trennmittels als nicht verfilmende Endschicht. Der Auftrag erfolgt als Pulver (in konzentrierter Form, 90 - 100%-ig) oder aus wäßriger Suspension mit 5 - 30 % Feststoffgehalt durch Sprühen. Die notwendige Menge ist niedriger als bei der Einarbeitung in die Polymerschicht und beträgt 0,1 - 2 % bezogen auf das Gewicht der Arzneiform.

• Pigmente:

Der Zusatz erfolgt nur selten in Form des löslichen Farbstoffs. In der Regel dispergiert man Aluminium- oder Eisenoxidpigmente. Titandioxid dient als Weißpigment . Übliche Einsatzmengen für Pigmente in den . erfindungsgemäßen Überzugs- und Bindemitteln zwischen 20 und 60 Gew.-%, bezogen auf die Polymermischung. Wegen des hohen Pigmentbindevermögens können jedoch auch Mengen bis zu 100 Gew.-% verarbeitet werden.

In einer besonders vorteilhaften Ausführungsform erfolgt der Einsatz des Pigments als nicht verfilmende Endschicht. Der Auftrag erfolgt als Pulver (in konzentrierter Form, 90 - 100%-ig) oder aus wäßriger Suspension mit 5 - 30 % Feststoffgehalt durch Sprühen. Die notwendige Menge ist niedriger als bei der Einarbeitung in die Polymerschicht und beträgt 0,1 - 2 % bezogen auf das Gewicht der Arzneiform.

Grundsätzlich müssen natürlich alle eingesetzten Substanzen toxikologisch unbedenklich und in Arzneimitteln ohne Risiko für Patienten zu verwenden sein.

Weitere Zuschlagstoffe können auch Weichmacher sein. Übliche Mengen liegen zwischen 0 und 50, bevorzugt 0 bis 20, insbesondere 0 bis 10 Gew.-%. Besonders bevorzugt sind allerdings höchstens 5 Gew.-% oder kein Weichmacher enthalten, da die Formulierungen durch die Anwesenheit der Komponenten (c) häufig bereits elastisch genug sind und zusätzlicher Weichmacher zu unerwünschter Klebrigkeit führen kann.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC), Acetyltriethylcitrat (ATEC) und Dibutylsebacat (DBS). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wäßriger Lösung oder nach thermische Vorbehandlung der Mischung vorgenommen werden. Auch können Mischungen von Weichmachern eingesetzt werden.

Das Herstellungsverfahren

Die Komponenten (a), (b) und (c) werden bei Raum- oder erhöhter Temperatur mit oder ohne Zusatz von Wasser und gegebenenfalls eines pharmazeutischen Wirkstoffs und der weiteren üblichen Zuschlagstoffe miteinander vermengt und das Überzugs- und Bindemittel durch Schmelzen, Gießen, Ausstreichen, Aufsprühen oder Granulieren hergestellt. Dabei ist die Verfilmung des Überzugs- und Bindemittels Voraussetzung für den funktionellen Effekt in Arzneiformen.

Die Verfilmung erfolgt, unabhängig von dem Auftragsverfahren durch Zuführung von Energie. Dies kann über Konvektion (Wärme), Strahlung (Infrarot oder Mikrowellen) oder Leitung erfolgen. Für den Auftrag als Suspensionsmittel eingesetztes Wasser verdampft dabei, gegebenenfalls kann auch ein Vakuum angewendet werden, um das Verdampfen zu beschleunigen.

Die für die Verfilmung notwendige Temperatur hängt von der Kombination der eingesetzten Komponenten ab.

Verwendung der erfindungsgemäßen Formulierung zur Herstellung von Bindemitteln:

Die Verwendung als Bindemittel erfolgt z. B. durch Aufsprühen der wäßrigen Polymersuspension auf wirkstofffreie Kerne (Nonpareilles) bei gleichzeitiger Zugabe von pulverförmigem Wirkstoffen oder deren Mischungen. Eine weitere Ausführungsform ist das Aufsprühen der wäßrigen Polymersuspension gemeinsam mit darin gelösten oder suspendierten Wirkstoffen.

Verwendung der erfindungsgemäßen Formulierung zur Herstellung von Überzugsmittel:

Vorgeformte Träger für die Überzüge sind Kapseln, Tabletten, Granulate, Pellets, Kristalle von regelmäßiger oder unregelmäßiger Form. Die Größe von Granulaten, Pellets oder Kristallen liegt zwischen 0,01 und 2,5 mm, die von Tabletten zwischen 2,5 und 30,0 mm. Kapsel bestehen aus Gelatine, Stärke oder Cellulosederivaten.

Pulver und Kristalle enthalten in der Regel 100 % der biologisch aktiven Substanz. Vorgeformte Träger enthalten von etwa 0,1 zu bis 99 % der biologisch aktiven Substanz bzw. des pharmazeutischen Wirkstoffs, sowie bis 1 bis 99,9 Gew.-%.weitere pharmazeutische Hilfsstoffe.

Übliche Herstellungsverfahren sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln.

Neben dem Wirkstoff können sie weitere pharmazeutische Hilfsstoffe enthalten: Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

Von besonderer Bedeutung ist die Zerfallszeit der Kerne, die die Freigabe des Wirkstoffs beeinflußt. Man strebt heute kurze Zerfallszeiten von unter 5, bzw. unter 10 min im Zerfallstest nach Ph. Eur. an. Längere Zerfallszeiten sind deswegen problematisch, weil zusätzliche Überzüge die Freigabe des Wirkstoffs weiter verzögern und den therapeutischen Effekt in Frage stellen können. Als Grenzwert wird heute eine Zerfallszeit von 30 min angesehen. Man testet in Wasser und künstlichem Magensaft (0,1 N HCl). Die eingesetzten Kerne sind homogen oder haben einen schichtartigen Aufbau. Sind Gravuren in die Oberflächen eingelassen, sollen diese durch Überzüge möglichst überdeckt aber wenig ausgefüllt werden. Die erfindungsgemäß eingesetzte Schichtdicke des Polymerpulvers variiert stark und hängt von dem Verarbeitungsverfahren oder der Menge an Zuschlagstoffen ab. Sie liegt zwischen 1 und 100 $\mu$m, bevorzugt zwischen 10 und 50 $\mu$m. Auf üblichen Tabletten entspricht das einem Polymerauftrag von 0,5 bis 5 Gew.-%.

Überzogene Mikropartikel können gemäß K. Lehmann et al., Drugs made in Germany 37, 2, 53-60 (1994) und T.E. Beckert et al, International Journal of Pharmaceutics 143,(1996), 13-23 zu zerfallenden Tabletten verpreßt werden, ohne signifikanten Einfluss auf die Funktion des Polymers.

Die Funktion der verfilmten Polymerschicht in der endgültigen Arzneiform kann vielfältig sein:

- Schutz vor schädlichen Umwelteinflüssen durch Feuchte, Gase, Licht usw.
- Geruchs- oder Geschmacksisolierung,
- Kennzeichnung durch Farbe
- Mechanische Stabilisierung
- Isolierung unverträglicher Inhaltsstoffe
- Vermeidung von Haftung an den Schleimhäuten.
- Zeitlich verzögerte Wirkstoffabgabe
- pH - gesteuerte Wirkstoffabgabe
- Isolierung von Kernen gegenüber weiteren Überzügen

Vorteilhaft ist die niedrige Viskosität der Polymermischung in wäßriger Dispersion auch bei hohen Feststoffanteilen bis zu 30 %, da Gravuren auf der Oberfläche von Tabletten detailliert nachgebildet werden. Besonders vorteilhaft ist die gute Schutz- und Isolierwirkung der erfindungsgemäßen Polymermischung bei gleichzeitig geringem Einfluß auf den Tablettenzerfall. Schon bei geringen Polymeraufträgen von 1 Gew.-% schon eine Geschmacksisolierung von mehr als 30 sec. erreicht. Dickere Überzüge mit einem Copolymer aus Methylmethacrylat, Butylmethacrylat, und Dimethylaminoethylmethacrylat in Verhältnis 25 : 25 : 50 (EUDRAGIT® EPO) verbessern die Geschmacksabdeckung, ohne jedoch die Zerfallszeit in 0,1N HCl zu verlängern. Ebenso vorteilhaft ist die zuverlässige Abdeckung gefärbter Kerne durch Überzüge mit hohem Pigmentanteil. Eine besondere Ausführungsform ist die Einbettung eines zweiten Wirkstoffs in den Überzug auf einen wirkstoffhaltigen Kern.

Auftrag auf der erfindungsgemäßen Formulierung zur Herstellung auf Träger

Die erfindungsgemäße Formulierung kann in Pulverform, als Schmelze oder in wäßriger Suspension durch Granulieren, Gießen, Ausstreichen oder mittels Sprühauftrag angewendet werden. Wasser dient dabei hauptsächlich als Vehikel, um dünne Umhüllungen gleichmäßig auf sphärische Kerne z. B. durch Sprühen aufzutragen. Für Beschichtungen werden außerdem Streichverfahren eingesetzt. Das eingesetzte Verfahren richtet sich hauptsächlich nach dem gewählten Träger. Trockene Pulver werden durch Ausstreichen oder Bestäuben aufgetragen, ggf. auch unter Einsatz elektrostatische Kräfte. Die Verfilmung kann durch Einwirkung von Wärme erfolgen. Für die Ausführung ist dabei entscheidend; dass gleichmäßige, geschlossene Schichten entstehen.

Auftragsverfahren gemäß dem Stand der Technik s. z. B. Bauer, Lehmann, Osterwald, Rothgang, "Überzogene Arzneiformen" Wissenschaftliche Verlagsgesellschaft mbH Stuttgart, Kap 7, S.165 - 196

Für die Applikation relevante Eigenschaften, geforderte Tests und Spezifikationen sind in Arzneibüchern aufgelistet. Details sind den gängigen Lehrbüchem zu entnehmen, z. B.:

- Voigt, R. (1984): Lehrbuch der pharmazeutischen Technologie; Verlag Chemie Weinheim - Beerfield Beach/Florida - Basel.
- Sucker, H., Fuchs, P., Speiser, P.: Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

Biologisch aktive Substanzen:

Die im Sinne der Erfindung eingesetzten Arzneistoffe sind dazu bestimmt, am oder im menschlichen oder tierischen Körper Anwendung zu finden, um

1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.

4. Krankheitserreger, Parasiten oder körperfremde Stoffe abzuwehren, zu beseitigen oder unschädlich zu machen oder

5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Die erfindungsgemäße Formulierung eignet sich zur Verabreichung grundsätzlich beliebiger pharmazeutischer Wirkstoffe, die vorzugsweise in isolierte oder geschützter Form verabreicht werden können, wie Antidepressiva, Betarezeptorenblocker, Antidiabetika, Analgetika, Antiphlogistika, Antirheumatika,Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen-Darmtherapeutika, Migränemittel, Mineralstoffpraparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren.

Beispiele geeigneter Wirkstoffe sind Acarbose, , Nichtsteroidale Antirheumatia, Herzglykoside, Acetylsalicylsäure, Virustatika, Aclarubicin, Acyclovir, Cisplatin, Actinomycin, alpha- und beta-Sympatomimetika, (Allopurinol, Alosetron, Alprostadil, Prostaglandine, Amantadin, Ambroxol, Amlodipin, Methotrexat, S-Aminosa-licylsäure, Amitriptylin, Amlodipin, Amoxicillin, Anastrozol, Atenolol, Atorvastatin, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Celetoxib, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkomalkaloide, Dimenhydrinat, Dimethylsulfoxid, Dimeticon, Dipyridarnoi, Domperidon und Domperidanderivate, Donepzil, Dopamin, Doxazosin, Doxorubizin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Esomeprazol, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Galantamin, Gallopamil, Ganciclovir, Gemfibrozil, Gentamicin, Ginkgo, Johanniskraut, Glibenclamid, Hamstoffderivate als orale Antidiabetika, Glucagon, Glucosamin und Glucosaminderivate, Glutathion, Glycerol und Glycerolderivate, Hypothalamushormone, Goserelin, Gyrasehemmer, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolclerivate, Itraconazol, Ketoconazol,. Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat,Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate; Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Orlistat, Oseltamivir, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Rosiglitazon, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralion, Silikate, Simvastatin, Sitosterin, Sotalol, Spagluminsäure, -Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Tegaserod, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Anti6strogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valdecoxib, Valproinsäure, Vancomycin, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zanamivir, Zidovudin, Zolmitriptan, Zolpidem, Zoplicon, Zotepin und dergleichen.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

Beispiele besonders bevorzugter Wirkstoffe sind

Acetylsalicyläure, Carbenoxolon, Cefalotin, Epinefrin, Imipramin, Kaliumjodid, Ketoprofen, Levodopa, Nitrazepam, Nitroprussid, Oxitetracyclin-HCl, Promethazin Omeprazol oder andere Benzimidazolderivate und Streptomycin.

Applikationsformen:

Grundsätzlich können die beschriebenen Arzneiformen direkt durch orale Applikation zur Anwendung kommen. Die erfindungsgemäß hergestellten Granulate, Pellets, oder Partikeln können in Gelatinekapseln, Beutel (Sachets) oder in geeignete Mehrdosenbehälter mit Dosiereinrichtung abgefüllt werden. Die Einnahme erfolgt in fester Form oder suspendiert in Flüssigkeiten. Durch Verpressen erhält man aus, ggf. nach Zumischung weiterer Hilfsstoffe, Tabletten die nach der Einnahme zerfallen und die meist überzogenen Untereinheiten freisetzen. Denkbar ist ebenso die Einbettung von Agglomeraten in Polyethylenglykol oder Lipide zur Herstellung von Suppositorien oder vaginalen Arzneiformen. Überzogene Tabletten werden in Blister oder Mehrdosenbehälter verpackt und vom Patienten direkt vor der Einnahme entnommen.

Wirkstoffklassen und Substanzen, die oftmals bitteren Geschmack hervorrufen können und sich vorteilhaft mit dem erfindungsgemäßen Überzugs- und Bindemittel formulieren lassen sind z. B.:

Analgetika und Antirheumatika:

Paracetamol, Diclofenac, Aceclofenac, Ibuprofen, Ketoprofen, Flubiprofen, Levacetylmethadol, Oxycodon
Psychopharmaka:
Prometazine, Donepezil, Modafinil, Nefazodon, Reboxetin, Sertindol, Sertralin
Antibiotika:
Erythromicyn, Roxithromycin, Clarithromycin, Grepafloxacin, Ciprofloxacin, Levofloxacin, Sparfloxacin, Trovafloxacin, Nevirapin
Betablocker
Propanolol, Metoprolol, Bisoprolol, Nebivolol
Antidiabetika:
Metformin, Miglitol, Repaglinid
H1 Antihistaminika:
Diphenhydramin, Fexofenadin, Mizolastin
H2 Antihistaminika
Cimetidin, Nizatidin, Ticlopidin, Cetridin, Ranitidin,
Vitamine: Thiaminenitrate; Weitere: Chinidin-Sulfat, Amiloprilose-HCl, Pseudoephedrin-HCl, Sildenafil, Topiramat, Granisetron, Rebamipide, Chinin-HCl

BEISPIELE

Beispiele 1 bis 14:

Dispergierung bzw. Auflösung eines Copolymers aus Methylmethacrylat, Butylmethacrylat, und Dimethylaminoethylmethacrylat in Verhältnis 25 : 25 : 50 mit einer mittleren Teilchengröße von 15 μm (EUDRAGIT® EPO).

Herstellungsweise:

In ein Gefäß wird Vollentmineralisiertes Wasser vorgegeben anschließend die Komponenten (a), (b) und (c) gemäß Anspruch 1 unter Rühren mittels Flügefrührers zugegeben mit ca. 400 Umdrehungen/min gerührt. Die gesamte Herstellung wird mit dem Polarisationsmikroskop 400-fache Vergrößerung verfolgt.
Die Dispersion bzw. Lösung hat einen Trockensubstanz-Gehalt von 15%, und ist niedrigviskos.

Beispiel 15: Es wird ein Copolymer aus Methylmethacrylat, Butylmethacrylat, und Dimethylaminoethylmethacrylat in Verhältnis 25 : 25 : 50 (EUDRAGIT® E100) gelöst in Isopropanol/Aceton (6:4) verwendet. Copolymergehalt 12,5 Gew.-%.

Die unten aufgeführten Versuche in Tabelle 1 sind auf Ansatzgröße von 100 g berechnet, wobei die Zusätze auf die Copolymermenge bezogen werden.
Abkürzungen:

bez.a. = bezogen auf
RT = Raumtemperatur

Tabelle 1: Herstellung des Überzugs- und Bindemittels

| Beispiel Nr. | Komponente (b) [% bez. auf Copolymer] | Komponente (c) [% bez.a.Copolymer] | Dispergier-temperatur | Dispergier zeit [h] | Dispergierung vollständig | Eigenschaften der getrockneten Filme | WDD [g/m²/d] |
|---|---|---|---|---|---|---|---|
| 1. | 7 Na - Laurylsulfat | 15 Laurinsäure | RT | ca. 3 | ja | Flexibel, klar | 324 |
| 2. | 7 Na - Laurylsulfat | 15 Myristinsäure | RT | ca. 4 | ja | Flexibel, leicht getrübt | 324 |
| 3. | 7 Na - Laurylsulfat | 15 Palmitinsäure | RT | ca. 6 | ja | flexibel, leicht getrübt | 168 |
| 4. | 7 Na - Laurylsulfat | 15 Stearinsäure | RT | ca. 6 | ja | flexibel, leicht getrübt | 179 |
| 5. | 7 Na - Laurylsulfat | 20 Stearinsäure | RT | ca. 6 | ja | flexibel, leicht getrübt | 213 |
| 6. | 7 Na - Laurylsulfat | 30 Stearinsäure | RT | ca. 5 | ja | flexibel, leicht getrübt | 289 |
| 7. | 7 Na - Laurylsulfat | 30 Stearinsäure | RT bis 73°C | ca. 4 | ja | flexibel, leicht getrübt | 186 |
| 8. | 10 Na - Laurylsulfat | 10 Stearinsäure | RT | ca. 6 | ja | flexibel, leicht getrübt | 125 |
| 9. | 10 Na - Laurylsulfat | 15 Stearinsäure | RT | ca. 6 | ja | spröde, leicht getrübt | 96 |
| 10. | 7 Na - Laurylsulfat | 15 Laurylalkohol | RT | ca. 24 | ja | flexibel, klar | 264 |
| 11. | 10 Na - Laurylsulfat | 10 Laurylalkohol | RT | ca. 3 | ja | flexibel, klar | 112 |

Negative Beispiele

| Beispiel Nr. | Komponente (b) | Komponente (c) | Dispergier-temperatur | Dispergier zeit [h] | Dispergierung vollständig | Eigenschaften der getrockneten Filme | WDD [g/m²/d] |
|---|---|---|---|---|---|---|---|
| 12. | 7 Na - Laurylsulfat | 15 Sebacinsäure | RT | ca. 2 | ja | spröde Kristalle | 454 |
| 13. | 7 Na - Laurylsulfat | 15 Behensäure | RT | - | nein | - | - |

Stand der Technik

| Beispiel Nr. | Komponente (b) | Komponente (c) | | | | | WDD [g/m²/d] |
|---|---|---|---|---|---|---|---|
| 14. | 7 Na - Laurylsulfat | 15 Diethylsebakat | | | | | 444 |
| 15. | - | - | EUDRAGIT® E 12,5 aus organischer Lösung (Isopropanol-Aceton = 6:4) | | | | 336 |

Wasserdampfdurchlässigkeiten, Filmeigenschaften

Aus den hergestellten Dispersionen werden mittels einer 250 μm Rakel auf Kondensatorpapier Filme gezogen und bei RT über Nacht trocknen lassen. Die trockenen Filme mit einer Schichtdicke von 30-35 μm (Kondensatorpapier mit eingerechnet) werden nun nach DIN 53122 bei 23°C 85% rel. Feuchte auf Wasserdampfdurchlässigkeit untersucht. Je Dispersion werden 6 Tests parallel durchgeführt.

Beschreibung des Tests:

In eine plangeschliffene Prüfzelle aus Glas (Innendurchmesser 2 cm) werden 3 g Kieselgel, Körnung 1-3mm/ Fa. Merck, mit Feuchtigkeitsindikator eingewogen und bei 110°C 3 Stunden getrocknet. Die Schliffe der Prüfzelle wird mit Silikonpaste gefettet, so dass nach Auflage des Filmes ein luftdichter Abschluss gewährleistet ist. Die ausgewählten Filme aufgelegt und mit einem ebenfalls plangeschliffenem silikongefetteten Glasring abdecken. Durch diese nun entstandene Filmfläche

von 3,14 cm$^2$ wird nun die Wasserdampfdurchlässigkeit (WDD) bei 23°C, und 85 % rel. Feuchte über einen Zeitraum von 16 und 24 h gravimetrisch bestimmt.

$$WDD\ (g/m^2/d) = 24/\ t\ x\ \Delta\ m/A\ x\ 24$$

t  = Versuchsdauer in Stunden zwischen den Wägungen aus denen die Gewichtsdifferenz $\Delta m$ bestimmt wird, d. h. 18 - 2 = 16 oder 24 - 2 = 22

$\Delta m$  = durch die Folie diffundierte Wassermenge in g nach 16 Stunden und 22 Stunden; als Bezugswert gilt das nach 2 Stunden erhaltene Gewicht der Prüfzelle + Film.

A  = Prüffläche des Films in cm$^2$ = 3,14cm$^2$

Es wird der Mittelwert aus den Ergebnissen (16 und 22h) von mindestens 5 Prüfzellen errechnet.

Überzüge aus den Dispersionen obiger Beispiele

Die Sprühversuche wurden im Erweka - Dragierkessel mit 2,5 bis 3 kg Ansatzgröße Placebotabletten, zum Teil ausgetauscht durch Chinidinsulfattabletten oder Silikageltabletten durchgeführt. Der Polymerauftrag ist 2 bis 4 mg / cm$^2$.

Trennmittel wird überwiegend in der Dispersion mittels Ultra- Turrax ca. 15 min homogenisiert und anschließend mit einer Zweikanal - Sprühpistole Walter NBA Düse 1,2 mm, Sprühdruck von 0,8 bis 1 bar auf die rotierenden Tabletten aufgesprüht. Die Materialtemperatur kann zwischen 27 bis 34°C, während die Sprühgeschwindigkeit 2,2 bis 3 g/min/ kg betragen. Anschließen im 40°C Umluft- Trockenschrank über 4 h getrocknet.

Tabelle 2:

| Lauf. Nr. | Versuch nach Rezeptur Nr. | Trennmittel (%b. a. Copo) | mg Copo/cm$^2$ | Zerfallszeit (min) | | Geschmacks- isolierung |
|---|---|---|---|---|---|---|
| | | | | MS | H2O | |
| 16. | 4. | 42 Pigment | 2 | 2,1 | 5,3 | > 8 min |
| 17. | 4. | 50 Talkum | 2 | 1,3 | 3,6 | n. b. |
| | | | 4 | 1,7 | 7,3 | n. b. |
| 18. | 14. | 35 Mg-stearat | 2 | 1,7 | 17,2 | n. b. |
| | | | 4 | 2 | 21,5 | n. b. |
| 19. | 8. | 15 Mg-Stearat 15 Pigmente | 2 | 1,2 | 2,2 | > 6 min |
| 20. | 15. | 50 Talkum | 2 | 1,1 | >60 | n. b. |
| | | | 4 | 1,7 | > 60 | n. b. |
| 21. | 4. | 35 Mg-Stearat | 2 | 1,7 | 3,9 | n. b. |
| | | | 4 | 1,97 | 6,4 | n. b. |
| Copo=Copolymer | | | | | | |

Die Zerfallszeit wurde in Anlehnung nach der Methode des europäischen Arzneibuchs bestimmt.

EP 1 368 007 B1

Tabelle 3:

| Wasseraufnahme von Silikageltabletten im Klimaschrank bei 40°C und 75 % rel. Feuchte mit 4mg Copolymer/cm$^2$. | | | | | | |
|---|---|---|---|---|---|---|
| Rezeptur | Wasseraufnahme [%] | | | | | |
| | nach 1 h | nach 2 h | nach 4 h | nach 6 h | nach 10 h | nach 24 h |
| Nach Beispiel 20 | 0,48 | 1,07 | 2,16 | 3,04 | 4,80 | 9,06 |
| Nach Beispiel 18 | 0,67 | 1,62 | 2,71 | 3,79 | 5,93 | 11,19 |
| Nach Beispiel 21 | 0,34 | 0,69 | 1,36 | 1,92 | 3,11 | 6,55 |
| Vergleichswert: HPMC | 2,68 | 5,31 | 9,46 | 12,30 | 14,80 | 15,10 |
| Vergleichswert: Silikageltabletten (ohne Überzug) | 7,71 | 11,94 | 14,61 | 15,10 | 15,16 | 15,64 |

Die Wasseraufnahme wurde gravimetrisch bestimmt.

**Patentansprüche**

1. Verfahren zur Herstellung eines Überzugs- und Bindemittels für orale oder dermale Arzneiformen bestehend im wesentlichen aus

   (a) einem Copolymer, bestehend aus radikalisch polymerisierten C1- bis C4-Estern der Acryl- oder Methacrylsäure und weiteren (Meth)acrylat-Monomeren die funktionelle tertiäre Ammoniumgruppen aufweisen, wobei das Copolymer in Pulverform mit einer mittleren Teilchengröße von 1 - 40 µm vorliegt

   (b) 3 bis 15 Gew.-%, bezogen auf (a), eines Emulgators mit einem HLB-Wert von mindestens 14

   (c) 5 bis 50 Gew.-%, bezogen auf (a), einer $C_{12}$- bis $C_{18}$-Monocarbonsäure oder einer $C_{12}$- bis $C_{18}$-Hydroxyl-verbindung

   wobei die Komponenten (a), (b) und (c) mit oder ohne Zusatz von Wasser und gegebenenfalls unter Zusatz eines pharmazeutischen Wirkstoffs und weiterer üblicher Zuschlagstoffe miteinander vermengt oder vermischt werden und das Überzugs- und Bindemittel aus der Mischung durch Schmelzen, Gießen, Ausstreichen, Aufsprühen oder Granulieren hergestellt wird.

2. Verfahren nach Anspruch 1, **dadurch gekennzeichnet, daß** ein Trennmittel auf das Überzugs- und Bindemittel als nicht verfilmende Endschicht aufgebracht wird,

3. Verfahren nach Anspruch 1, **dadurch gekennzeichnet, daß** ein Pigment auf das Überzugs- und Bindemittel als nicht verfilmende Endschicht aufgebracht wird.

4. Überzugs- und Bindemittel für Arzneiformen, herstellbar nach einem oder mehreren der Ansprüche 1 bis 3.

5. Arzneiform enthaltend das Überzugs- und Bindemittel nach Anspruch 4 sowie einen pharmazeutischen Wirkstoff.

6. Arzneiform nach Anspruch 5, **dadurch gekennzeichnet, daß** eine feuchteempfindlicher pharmazeutischer Wirkstoff aus den Wirkstoffklassen der Analgesika, Antirheumatika, Wirkstoffe zur Behandlung von Magengeschwüren, Antibiotika, Antihypotonika, Antidepressiva Schilddrüsentherapeutika, Anti Parkinson-Wirkstoffe, Anxiolytika oder Neuroleptika enthalten ist.

7. Arzneiform nach Anspruch 6, **dadurch gekennzeichnet, daß** als feuchteempfindlicher pharmazeutischer Wirkstoff Acetylsalicyläure, Carbenoxolon, Cefalotin, Epinefrin, Imipramin, Kaliumjodid, Ketoprofen, Levodopa, Nitrazepam, Nitröprussid, Oxitetracyclin-HCl, Promethazin, Omeprazol oder andere Benzimidazolderivate oder Streptomycin enthalten ist.

**8.** Verwendung des Überzugs- und Bindemittels nach Anspruch 4 als feuchtigkeitsisolierender Überzug für wirkstoffhaltige pharmazeutische Zusammensetzungen.

**9.** Verwendung des Überzugs- und Bindemittels nach Anspruch 4 als geschmacksisolierender Überzug für wirkstoffhaltige pharmazeutische Zusammensetzungen.

**Claims**

**1.** Process for preparing a coating and binding agent for oral and dermal pharmaceutical preparations, consisting essentially of

(a) a copolymer, consisting of radically polymerised $C_1$-$C_4$-esters of acrylic or methacrylic acid and other (meth) acrylate monomers which comprise functional tertiary ammonium groups, the copolymer being in powder form with an average particle size of 1 - 40 $\mu$m,

(b) 3 to 15 wt.%, based on (a), of an emulsifier with an HLB value of at least 14,

(c) 5 to 50 wt.%, based on (a), of a $C_{12}$-$C_{18}$-monocarboxylic acid or a $C_{12}$-$C_{18}$-hydroxyl compound,

the components (a), (b) and (c) being combined or mixed together with or without the addition of water and optionally with the addition of a pharmaceutical active substance and other conventional additives, and the coating and binding agent being prepared from the mixture by melting, casting, spreading, spraying or granulating.

**2.** Process according to claim 1, **characterised in that** a parting compound is applied to the coating and binding agent as a non-film-forming final layer.

**3.** Process according to claim 1, **characterised in that** a pigment is applied to the coating and binding agent as a non-film-forming final layer.

**4.** Coating and binding agent for pharmaceutical preparations, which can be produced according to one or more of claims 1 to 3.

**5.** Pharmaceutical preparation containing the coating and binding agent according to claim 4 as well as a pharmaceutical active substance.

**6.** Pharmaceutical preparation according to claim 5, **characterised in that** it contains a moisture-sensitive pharmaceutical active substance selected from among the active substance categories of analgesics, antirheumatics, active substances for treating stomach ulcers, antibiotics, antihypotensives, antidepressants, thyroid treatment agents, anti-Parkinson's active substances, trariquillisers or neuroleptics.

**7.** Pharmaceutical preparation according to claim 6, **characterised in that** it contains as moisture-sensitive pharmaceutical active substance acetylsalicylic acid, carbenoxolone, cefalothin, epinephrine, imipramine, potassium iodide, ketoprofen, levodopa, nitrazepam, nitroprusside, oxytetracyclin-HCl, promethazine, omeprazole or other benzimidazole derivatives or streptomycin.

**8.** Use of the coating and binding agent according to claim 4 as a moisture-proof coating for pharmaceutical compositions containing active substances.

**9.** Use of the coating and binding agent according to claim 4 as a flavour-insulating coating for pharmaceutical compositions containing active substances.

**Revendications**

**1.** Procédé de préparation d'un agent d'enrobage et liant pour formes pharmaceutiques orales ou dermales comportant essentiellement :

(a) un copolymère constitué d'esters en $C_1$ à $C_4$ polymérisés par voie radicalaire de l'acide acrylique ou méthacrylique et d'autres monomères de (méth)acylates qui portent des groupes ammonium tertiaires fonctionnels, le copolymère se présentant sous forme de poudre d'une granulométrie moyenne de 1 - 40 µm,

(b) 3 à 15 % en poids, rapporté à (a), d'un émulsionnant présentant un indice HLB d'au moins 14,

(c) 5 à 50 % en poids, rapporté à (a), d'un acide monocarboxylique en $C_{12}$ à $C_{18}$ ou d'un composé hydroxylé en $C_{12}$ à $C_{18}$,

en mélangeant les composants (a), (b) et (c) avec ou sans addition d'eau et, le cas échéant, avec addition d'une substance active pharmaceutique et d'autres substances additives courantes, puis en préparant l'agent d'enrobage et liant à partir du mélange par fusion, coulée, étalement, pulvérisation ou granulation.

2. Procédé selon la revendication 1,
**caractérisé en ce qu'**
on applique un agent de séparation sur l'agent d'enrobage et liant comme couche finale non filmogène.

3. Procédé selon la revendication 1,
**caractérisé en ce qu'**
on applique un pigment sur l'agent d'enrobage et liant comme couche finale non filmogène.

4. Agent d'enrobage et liant pour formes pharmaceutiques, pouvant être préparé selon une ou plusieurs des revendications 1 à 3.

5. Forme pharmaceutique contenant l'agent d'enrobage et liant selon la revendication 4, ainsi qu'une substance active pharmaceutique.

6. Forme médicamenteuse selon la revendication 5,
**caractérisée en ce qu'**
elle contient une substance active pharmaceutique sensible à l'humidité, appartenant aux classes de substances actives des analgésiques, antirhumatiques, substances actives pour le traitement des ulcères de l'estomac, antibiotiques, antihypotoniques, antidépresseurs, thérapeutiques de la glande thyroïde, substances actives anti-parkinson, anxiolytiques, ou neuroleptiques.

7. Forme médicamenteuse selon la revendication 6,
**caractérisée en ce qu'**
elle contient comme substance active pharmaceutique sensible à l'humidité, l'acide acétylsalicylique, la carbonoxolone, la céfalotine, l'épinefrine, l'imipramine, l'iodure de potassium, le cétoprofène, la lévodopa, le nitrazepame, le nitroprusside, l'oxytétracycline-HCl, la prométhazine, l'oméprazole et d'autres dérivés de benzimidazoles ou la streptomycine.

8. Utilisation de l'agent d'enrobage et liant selon la revendication 4, comme revêtement isolant vis-à-vis de l'humidité pour préparations pharmaceutiques contenant des substances actives.

9. Utilisation de l'agent d'enrobage et liant selon la revendication 4, comme revêtement isolant vis-à-vis du goût pour préparations pharmaceutiques contenant des substances actives.